# EUROPEAN PATENT APPLICATION

(11) **EP 3 020 816 A1**
(43) Date of publication of application: **18.05.2016**
(21) Application number: 14192686.5
(22) Date of filing: 11.11.2014
(51) Int. Cl.: C12N 15/63, C12N 15/87

(54) **Bacterial mediated gene therapy**

(71) Applicant: University College Cork, Cork City (IE)
(72) Inventor: Tangney, Mark, Cork (IE); Byrne, William, Cork (IE)
(74) Representative: Purdy, Hugh Barry

(57) **Abstract**

A live non-invasive bacteria comprises a transgene under the control of a promotor, wherein the transgene encodes a phagocytic cell-relevant therapeutically active agent and ideally a tumor associated macrophage (TAM) relevant therapeutically active agent in the form of an effector molecule capable of re-programming a tumor associated macrophage (TAM) from a pro-disease phenotype to an anti-disease phenotype. Use of the live non-invasive bacterium in a method of treating a phagocytic cell relevant disease, especially cancer, in a mammal is also disclosed.

## Description

### Introduction

Phagocytic cells such as macrophages, dendritic cells (DCs) and neutrophils fulfil essential roles in homeostasis, tissue repair and immunity. However they are also known to play a negative role, either aetiologically or pathophysiologically, in several diseases. Elevated circulating levels along with enhanced recruitment of such cells to inflamed tissues has been observed in a number of chronic inflammatory conditions including inflammatory bowel disease (IBD), rheumatoid arthritis (RA) and multiple sclerosis (MS). In active IBD, neutrophil accumulation in the intestinal lumen correlates with epithelial injury and clinical disease activity. Importantly manipulating phagocytic cells has proven therapeutically beneficial; neutrophil depletion provided promising results in IBD clinical trials. Similarly, targeting immunogenic DCs by blocking their persistent activation of self-reactive T cells with a monoclonal antibody yielded positive clinical benefits in patients with RA, IBD and psoriasis.

In cancer, phagocytic cells are often especially problematic. Macrophages perpetuate a cycle of chronic inflammation that provokes cancer initiation. They facilitate angiogenesis, tumour cell migration, invasion and intravasation, all leading to metastatic disease. Strategies to manipulate tumour-associated macrophages have shown promise in pre-clinical cancer models and in clinical trials. The role of tumour associated neutrophils (TANs) is somewhat unclear with their prognostic significance in human cancer remaining controversial. Pro-tumoural functions of TANs have been reported to include angiogenesis, blockade of anti-tumoural immune responses and promotion of tumour cell proliferation, invasion and spread. Tumour-promoting DCs have been identified in both breast and pancreatic cancer. DC maturation is subverted by the tumour which skews the immune response towards a Th2 phenotype and production of tumour- promoting cytokines.

Interest in phagocytic cells as disease targets reinforces the requirement for novel, selective delivery strategies to reach these cells.

Hagemann¹ first proposed a strategy and validated the therapeutic effect of treating ovarian cancer by re-educating ascitic macrophages. To re-educate ascitic macrophages, the Hagemann study isolated TAM from the ascitic fluid of mice, grew them in the lab, placed the gene in the cells *ex vivo* (utilising traditional chemical methods), and then introduced these newly re-educated cells into mice. The therapeutic effects were very powerful. However, this *ex vivo* method of transfecting TAM is very artificial and impractical/expensive in a clinical setting, precluding commercial/clinical exploitation of the strategy.

It is an object of the invention to overcome at least one of the above-referenced problems.

### Statements of Invention

The invention addresses at least one of the problems of the prior art by providing gene therapy of diseases characterised by macrophage accumulation (i.e. solid tumor cancer and malignant ascites) in which a non-invasive bacterium is employed as a delivery vehicle. Use of a non-invasive bacterium as a delivery vehicle selectively targets the transgene to disease-associated macrophage (and other phagocytic cells), where the transgene is expressed by the non-invasive bacterium or passively transfects the macrophage. The transgene typically encodes an effector molecule capable of re-programming the disease associated macrophage from a pro-disease phenotype to an anti-disease phenotype, or an effector molecule that directly targets disease cells to attenuate or inhibit the disease pathology. Using an *in vitro*-differentiated human monocyte cell line and two *in vivo* mouse models (an ovarian cancer ascites and a solid colon tumour model) proof of delivery is demonstrated with bacteria carrying reporter constructs and therapeutic constructs. The data demonstrate the ability to specifically transfect (deliver genes to) phagocytic cells within the malignant peritoneum in a mouse model, the ability to specifically transfect (deliver genes to) phagocytic cells within solid tumours in a mouse model, and demonstrates an improvement in the immune environment at the cancer locus, as a result of delivery of a therapeutic gene in subcutaneous tumour mouse models.

In a first aspect, the invention provides a live non-invasive bacterium comprising a transgene, typically under the control of a promotor, wherein the transgene encodes a phagocytic cell-relevant therapeutically active agent.

Preferably, the transgene encodes a tumor associated macrophage (TAM) relevant therapeutically active agent.

Preferably, the phagocytic cell relevant therapeutically active agent comprises an effector molecule capable of re-programming a disease-associated phagocytic cell from a pro-disease phenotype to an anti-disease phenotype.

Preferably, the phagocytic cell relevant therapeutically active agent comprises an effector molecule capable of re-programming a disease-associated macrophage cell from a pro-disease phenotype to an anti-disease phenotype.

Preferably, the phagocytic cell relevant therapeutically active agent comprises an effector molecule capable of re-programming a tumor-associated macrophage cell from a pro-disease phenotype to an anti-disease phenotype.

Suitably, the effector molecule comprises a macrophage NFκB pathway attenuator..

Typically, the effector molecule comprises a mediator that modulates host cell cytokine profile (for example by inducing a pro-inflammatory response (in the case of treatment of cancer) or inhibiting a pro-inflammatory response (in the case of an inflammatory disease such as cardiovascular disease or rheumatoid arthritis)). Preferably, the effector molecule comprises a mediator that induces expression of a pro-inflammatory cytokine (for example IL-1, IFNγ or TNFα. In another embodiment, the effector molecule comprises a mediator that inhibits expression of a pro-inflammatory cytokine (for example IL-1 or TNFα).

In one embodiment, the tumor associated macrophage (TAM) relevant therapeutically active agent comprises an effector molecule capable of directly targeting a cancer cell.

Preferably, the effector molecule is a monoclonal antibody.

Suitably, the promotor is configured to mediate expression in a eukaryotic cell. Preferably, the promotor is a eukaryotic promotor.

Typically, the therapeutically active agent is selected from a protein, a peptide, an oligopeptide, a polypeptide, and a low molecular weight gene expression inhibitor or activator. Typically, the low molecular weight gene expression inhibitor or activator is a nucleic acid, preferably an RNA molecule. Examples include siRNA, shRNA, saRNA, miRNA or a ribozyme. Preferably, the effector molecule capable of re-programming a disease associated macrophage from a pro-disease phenotype to an anti-disease phenotype is a low molecular weight gene expression inhibitor or activator, preferably an RNA molecule. Preferably, the effector molecule capable of re-programming a tumor associated macrophage (TAM) from a pro-disease phenotype to an anti-disease phenotype is a low molecular weight gene expression inhibitor or activator, preferably an RNA molecule.

The invention also provides a live non-invasive bacterium according to the invention, for use in a method of treating a disease in a mammal characterised by phagocytic cell accumulation at a disease locus in the mammal, wherein the phagocytic cell-relevant therapeutically active agent is specific for the disease.

The invention also relates to a method of treating a disease in a mammal characterised by phagocytic cell accumulation at a disease locus in the mammal comprising a step of administering to the mammal a live non-invasive bacterium according to the invention, wherein the phagocytic cell-relevant therapeutically active agent is specific for the disease. Preferably, the live non-invasive bacterium according to the invention is administered locally to a disease locus in the mammal.

Preferably, the disease is cancer. Typically, the disease is selected from a solid tumor and malignant ascites.

Suitably, the disease is cancer, and wherein the phagocytic cell-relevant therapeutically active agent comprises an effector molecule capable of re-programming a tumor associated macrophage (TAM) from a pro-tumorigenic phenotype to an anti-tumor phenotype.

Typically, the effector molecule is a low molecular weight inhibitor of macrophage NFκB pathway or a modulator of host cell cytokine expression.

In another embodiment, the disease is cancer, and wherein the phagocytic cell-specific therapeutically active agent comprises an effector molecule capable of directly targeting a cancer cell.

Typically, the effector molecule is a protein or peptide drug, for example monoclonal antibody.

In one embodiment of the invention, the cancer is a solid tumor cancer, and in which the live non-invasive bacteria is administered intratumorally.

In another embodiment of the invention, the cancer is a solid tumor cancer, and in which the live non-invasive bacteria is administered intravenously.

In another embodiment, the cancer is a peritoneal cancer, and in which the live non-invasive bacteria is administered intraperitoneally.

### Brief Description of the Figures

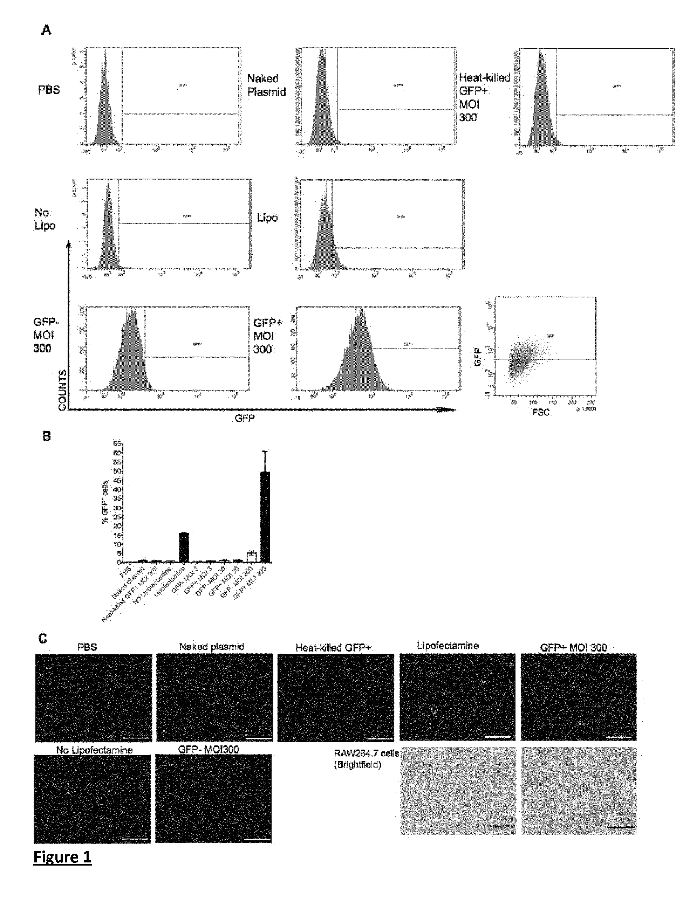

**Figure 1****: Vector delivery** *in vitro.* RAW264.7 macrophages were exposed for 5 H to *E.coli MG1655* carrying a GFP plasmid under a eukaryotic promoter at different multiplicities of infection. Macrophages were incubated for 22 H prior to flow cytometric analysis of viable cells to determine the number of GFP⁺ cells. (A) Representative histograms showing the percentage GFP⁺ RAW264.7 cells. The appropriate gating controls for each condition appear in the far left column. (B) Graphical representation of the flow cytometry analysis. Data show the average number of GFP⁺ cells +/- SEM of two independent replicates. (C) Representative fluorescent images, along with the corresponding brightfield image underneath showing the green GFP⁺ macrophages in the Lipofectamine and GFP⁺ MOI 300-treated cells compared with their respective negative controls. Original magnifications, x400. Scale bar is 50µm.

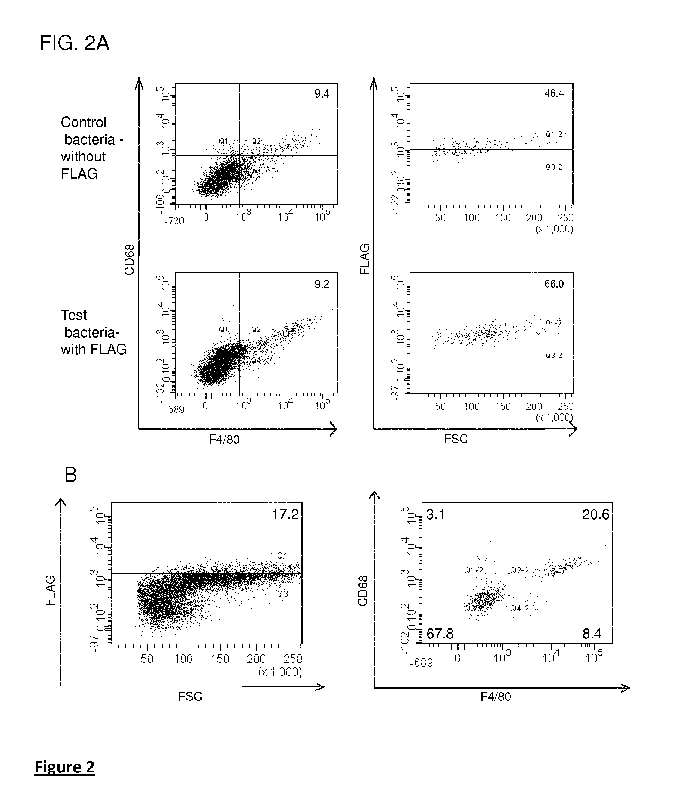

**Figure 2****: Vector delivery to CT26 TAMs 48 H post administration.** *E.coli MG1655* +/-FLAG-tagged plasmid were i.t. administered directly to CT26 tumours. After 48 H tumours were harvested and individual cells analysed for tumour-associated macrophage (TAM) transfection by FACS detection of FLAG. **(A) % FLAG⁺ TAM:** CD68⁺F4/80⁺ TAMs (left) were gated on FLAG positivity (right). The top two panels refer to data from mice that received *E. coli* lacking a FLAG plasmid (-FLAG), while the lower 2 panels refer to data from mice that received *E*. *coli* bearing the FLAG plasmid (+FLAG). **(B) % FLAG⁺ which are TAM;** FLAG⁺ cells (left) were gated on macrophage markers (CD68 and F4/80) (right). Each panel shows data from a single representative animal.

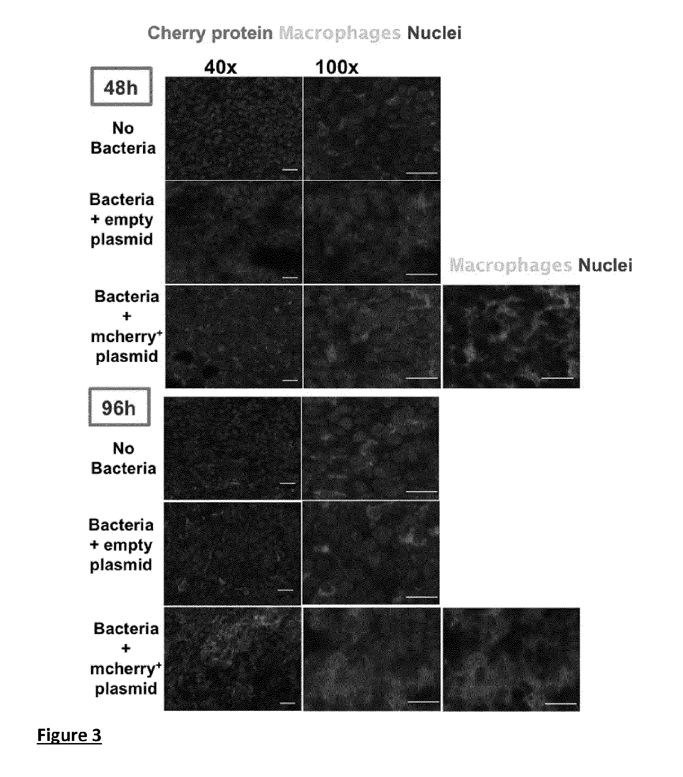

**Figure 3****: Bacterial-mediated gene delivery to solid tumour macrophages *in vivo.*** Mice bearing s.c. CT26 tumours were I.T. injected with PBS or 1 X 10⁶ *E. coli* MG1655 with or without the mCherry fluorescent protein under transcriptional control of a eukaryotic promoter.
Immunofluorescently-stained tumour sections obtained from 2-3 individual mice per group at 48 H and 96 H were analysed by fluorescence microscopy. (A) Frozen tumour sections (5µm) were stained for F4/80⁺ cells (green) and co-stained for nuclei (blue). mCherry was detected using native protein fluorescence (red). mCherry expression was found to co-localise with macrophages in the tumour tissue. A representative picture for each group is shown. Original magnifications, x400, x100. Scale bar is 20µm.

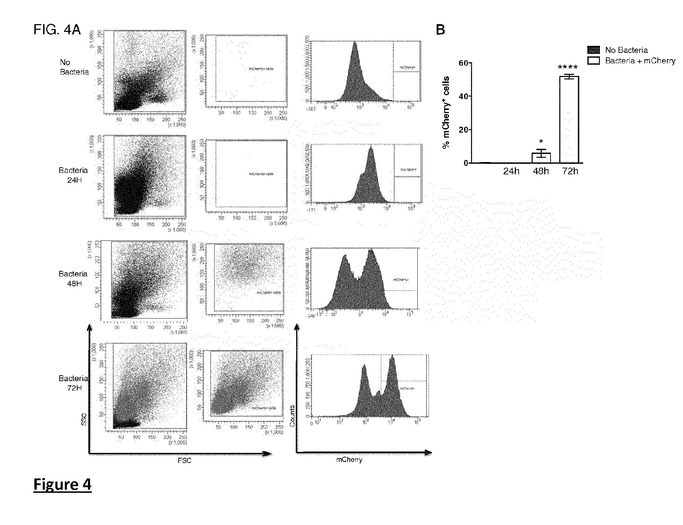

**Figure 4****: Vector delivery of mCherry to host ascitic cells 24, 48, 72 H post administration.** The cells were harvested from the ascites and analysed for mCherry expression by flow cytometry, in which 100,000 events were recorded. (A) Representative dot plots and histograms showing the percentage of mCherry⁺ host cells in the ascites. **(B)** Graphical representation of flow cytometry analysis. Data represent the mean percentage of mCherry⁺ cells at each time point ± SEM of 1-3 individual mice per group per time point.*P < 0.05, **P < 0.01, ***P < 0.001, ****P < 0.0001.

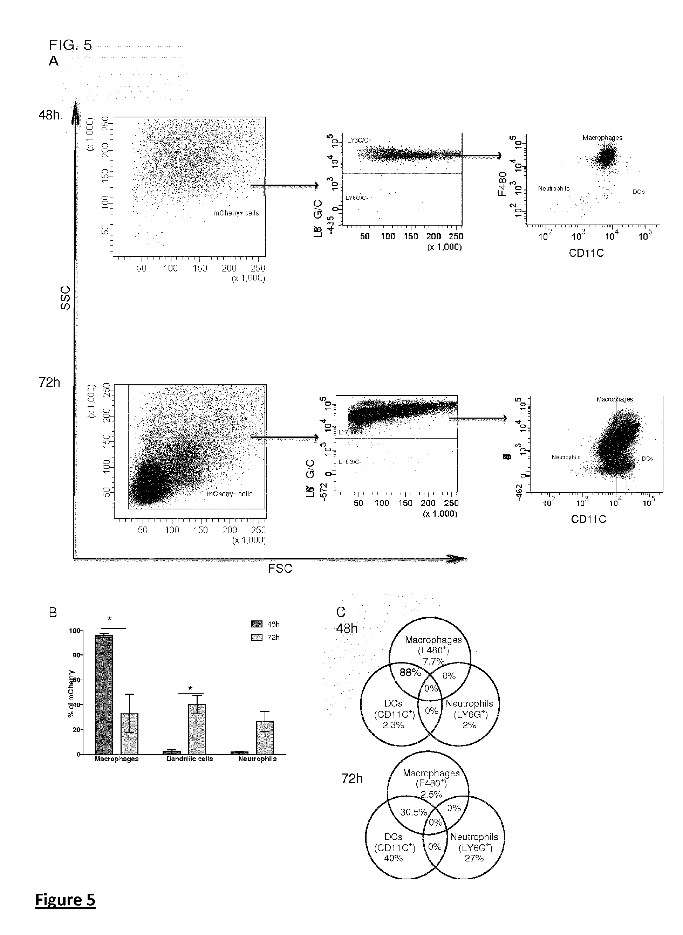

**Figure 5****: Bacterial-mediated gene delivery is specific to phagocytic cells.** Phenotype of the mCherry⁺ cells in the ascites. Single cell suspensions were prepared from ascites harvested from albino C57BL/6 mice at different time points post bacterial administration. The cells were stained with flurochrome-labelled mAb and analysed by flow cytometry in which 100,000 events were recorded. (A) Representative dot plots showing the phenotype of mCherry⁺ cells in the ascites at 48 and 72 H. Live cells were first gated on mCherry. All mCherry positive cells were LY6G/C+ (i.e. phagocytes). These cells were then gated individually on the phagocytic cell markers F480, CD11C and LY6G. (B) Data represent the mean percentage of F4/80⁺ (Macrophages), CD11C⁺ (DCs) and Ly6G⁺ (Neutrophils) mCherry⁺ cells and is expressed as the mean ± SEM of 2-3 individual mice per group per time point. *P < 0.05, **P < 0.01, ***P < 0.001, ****P < 0.0001. (C) Venn diagram showing breakdown of ascites cells expressing mCherry at 48 H and 72 H post bacterial administration. F480+LY6G/LY6C+CD11C⁺ are presented as macrophages, F480-LY6G/LY6C⁺CD11C⁺ as DCs and F480⁻CD11C⁻LY6G/LY6C⁺cells as neutrophils/ granulocytes. Data are expressed as the mean of 2-3 individual mice per time point.

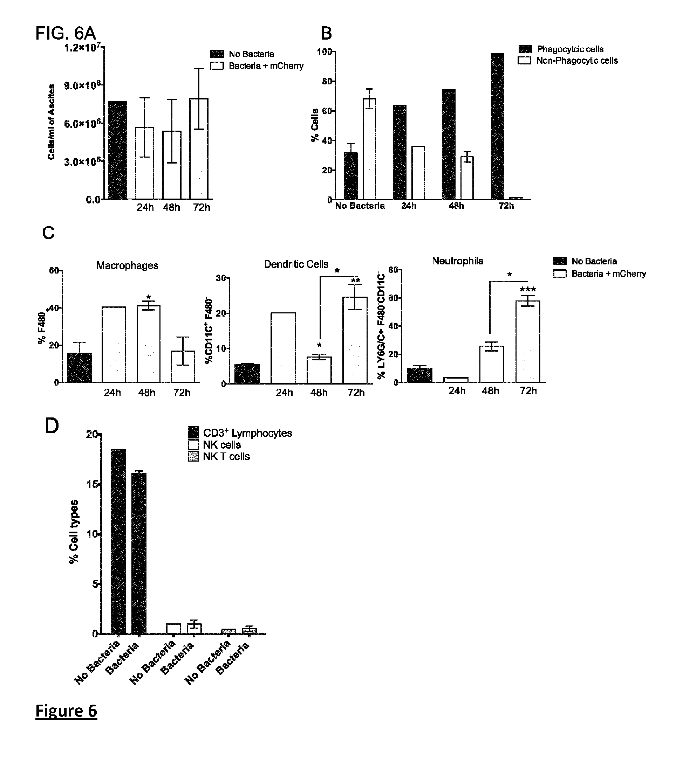

**Figure 6****: Bacterial vector leads to phagocytic cell differentiation.** Cells were harvested from the ascites at 24, 48 and 72 H post bacterial administration and counted. (A) Total number of cells per ml of ascites fluid recovered at the various time points post bacterial administration. Counted cells were then stained with fluorochrome-labelled antibody and analysed by flow cytometry in which 100,000 events were recorded. (B) Data represent the mean percentage of LYG/C⁺ (phagocytic) and LY6G/C⁻ (non-phagocytic cells) in the ascites and is expressed as the mean ± SEM of 1-3 individual mice per group. (C) Data represent the mean percentage of F4/80 (macrophages), CD11c (DC) and Ly6G (neutrophils) positive cells present in the ascites at each time point and is expressed as the mean ± SEM of 1-3 individual mice per group per time point. *P < 0.05, **P < 0.01, ***P < 0.001. (D) Percentage of non-phagocytic cells in ascites at 48 H post administration of bacteria. Data represent the mean percentage of CD3 (T cells), DX5 (NK) and CD3DX5 (NKT) positive cells present in ascitic fluid and are expressed as the mean ± SEM of 1-3 individual mice per group.

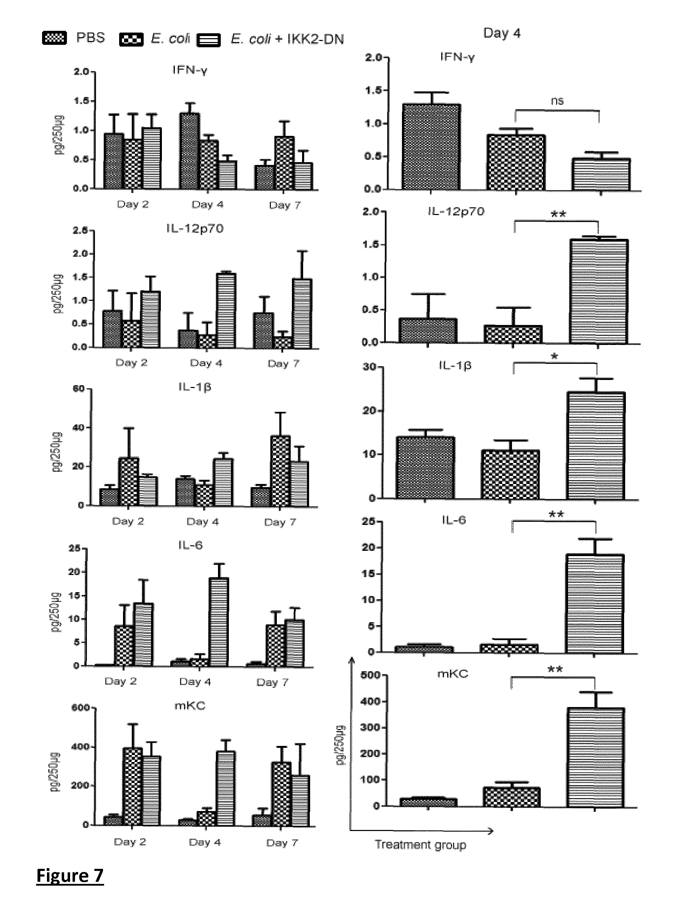

**Figure 7** **Cytokine profile of solid tumours following bacterial delivery of IKK2-DN.**

Balb/C mice bearing s.c. CT26 tumours were treated with PBS, *E.coli* or *E.coli* + IKK2-DN plasmid. The cytokine profile within the tumour was determined using a mouse pro-inflammatory multiplex plate. Levels are expressed as the mean +/- SEM for 3 mice per group. The left panel displays individual cytokine profiles at three distinct time points post bacterial administration. The right panel focuses on Day four where the greatest change in cytokine levels was detected. Statistics compare the two bacterial treated groups where one group received the therapeutic IKK2-DN gene and the other did not. *indicates p< 0.05. **indicates p< 0.005.

### Detailed Description of the Invention

### Definitions:

"Non-invasive bacterium" means a bacterium that cannot actively mediate its internalisation to a eukaryotic cell, lacks invasion factors, and is non-pathogenic and non-virulent, but is capable of being taken up by a phagocytic cell. Examples of non-invasive bacteria include non-pathogenic strains *of E. coli,* fastidious Gram negative bacilli, Enterobacteriaceae, non-glucose fermenting Gram negative bacilli, glucose fermenting oxidase-positive Gram negative bacilli; non-pathogenic Lactic Acid Bacteria such as bifidobacteria, lactobacilli and non-pathogenic Gram positive cocci such as non-pathogenic members of the genera *Streptoccocus* and *Lactococcus;* Non-pathogenic members of Gram-positive genera such as *Listeria* (e.g. *L. welshimeri);* Strains of Gram negative or Gram positive bacteria rendered non-invasive by genetic modification (e.g. species of *Salmonella* rendered non-invasive by deletion of relevant virulance factors).

"Transgene" means an exogenous gene or genetic material that is transferred into the non-invasive bacteria by means of a genetic engineering technique, and which is capable of being expressed by the non-invasive bacteria or a phagocytic cell targeted by the non-invasive bacteria. Examples of transgenes includes nucleic acids encoding for proteins, peptides, oligopeptides, polypeptides, mRNA capable of interfering with host cell transcription, low molecular weight inhibitors of gene expression, including siRNA, shRNA, miRNA, ribozymes, or activators of gene expression such as small activating RNA (saRNA).

"Under the control of a promotor" means that the transgene is operably linked to the promotor such that the promotor is capable of driving expression of the transgene. Typically, the transgene and promotor are provided in the form of a suitable vector, for example a plasmid comprising the transgene and the promotor. Examples of suitable vectors include any plasmid capable of being maintained by the relevant bacterial cell (e.g. features a suitable origin of replication, such as of the pUC, pAMbeta type etc.) with the capacity to introduce and maintain heterologous DNA sequences within the plasmid (such as within a multiple cloning site). The heterologous DNA may feature a promotor suitable for mediating expression of the sequence relevant to the therapeutic strategy (see transgene).

"Promotor" means a nucleic acid operably connected to a transgene and capable of driving expression of the transgene in either the bacteria or a host phagocytic cell. The promotor may be a prokaryotic promotor (a promotor capable of driving expression of the transgene using the genetic machinery of the non-invasive bacteria) or a eukaryotic promotor (a promotor capable of driving expression of the transgene using the genetic machinery of a eukaryotic host cell).

"Phagocytic cell-relevant" as applied to a therapeutically active agent means an agent that is therapeutically active against a disease characterised by phagocytic cell accumulation at the site of the disease. Examples of such diseases (i.e. "phagocytic cell-relevant diseases") include cancer (especially solid tumors and malignant ascites), cardiovascular disease, and rheumatoid arthritis, all of which are characterised by macrophage accumulation at a disease locus. Typically, the phagocytic cell-relevant disease is a macrophage cell relevant disease.

"Therapeutically active agent" means an agent that when administered to a mammal exhibiting a phagocytic cell-relevant disease is capable of attenuating or inhibiting disease pathology or progression by means of (a) directly targeting the disease cells to attenuate or inhibit disease pathology (for example a therapeutic monoclonal antibody that targets cancer cells), or (b) re-programming a phagocytic cell (typically a macrophage) from a pro-disease phenotype to an anti-disease phenotype (examples include inhibiton of the macrophage NFκB pathway, or a mediator that modulates host cell cytokine profile). Preferably, the phagocytic cell-specific therapeutically active agent is a macrophage-specific therapeutically active agent.

"Non-immunogenic" as applied to a therapeutically active agent means a therapeutically active agent that does not. present to the host immune system as an antigen.

"Tumor associated macrophage (TAM) relevant therapeutically active agent" means an agent that when administered to a mammal with a cancer characterised by accumulation of macrophages at a cancer locus is capable of attenuating or inhibiting disease pathology or progression by means of (a) directly targeting the cancer cells to attenuate or inhibit disease pathology or progression (for example a therapeutic monoclonal antibody that targets cancer cells), or (b) re-programming a tumor-associated macrophage from a pro-disease phenotype to an anti-disease phenotype (examples include an inhibitor of the macrophage NFκB pathway, or a mediator that modulates host cell cytokine profile).

"Therapy" or "treatment" should be taken to mean a course of action / dosing regime that either inhibits, delays or prevents the progression of disease.

"Phagocytic cell" means a cell that protects the body by ingesting foreign matter, and includes white blood cells such as neutrophils, monocytes, macrophages and mast cells, and dendritic cells. Preferably, the phagocytic cell is a macrophage.

"Disease-associated macrophage" means a macrophage that accumulates at a disease locus, and would include cancer-associated macrophage, coronary disease associated macrophage, and arthritis-associated macrophage. Examples of specific cancer associated macrophage include tumor-associated macrophage (TAM) that are known to accumulate in cancer tumors, especially solid tomors, and cancer cell associated macrophage (for example macrophage that accumulate at a site of malignant ascites).

"Pro-disease phenotype" as applied to a disease-associated macrophage means a phenotype that contributes to the disease pathology, generally by means of modulating the macrophages' immune response to the disease. Tumor-associated macrophage (TAMs) generally follow an "M2" phenotype and play a detrimental role in cancer pathophysiology, the cancer-associated macrophage exhibiting a pro-tumorigenic phenotype by modifying the immune response to tolerate tumor cells, promoting cancer cell motility, and encourage metastasis and stimulate angiogenesis. Conversely, inflammatory diseases, such as arthritis, associated with excessive local pro-inflammatory macrophage activity (of the "M1" phenotype) play a detrimental role in disease pathophysiology.

"Anti-disease phenotype" as applied to a disease-associated macrophage means a phenotype that attenuates or inhibits the pathology of the disease, generally by means of modulating the macrophages' immune response to the disease. When the disease is cancer, the anti-disease phenotype means that the macrophages pro-disease phenotype is attenuated or inhibited. In one example, the phenotype of the TAM is modified such that tolerance to tumor cells is switched off, or that the macrophage mediates an anti-tumor immune response.

"Effector molecule" means a molecule that is capable of re-programming a disease associated macrophage from a pro-disease phenotype to an anti-disease phenotype. Example of such molecules are proteins, peptides (including oligopeptides and polypeptides), and low molecular weight inhibitors of expression of specific genes. In the context of protein-based therapeutics for cancer, the effector molecule may be selected from a therapeutic antibody, a cytokine, or an enzyme inhibitor therapeutic.

"Therapeutic antibody" means an antibody, generally a monoclonal antibody, that has a therapeutic effect in a mammal. Examples of therapeutic antibodies indicated for cancer include Catumaxomab (indicated for treatment of malignant ascites), and Bevacizumab (indicated for treatment of metastatic cancers).

"Enzyme inhibitor therapeutic" means an enzyme inhibitor that is indicated for the treatment of one or more cancers. Examples include tyrosine kinase inhibitors (TKI) for example Sunitinib (indicated for renal cell carcinoma), Imatinib (indicated for leukaemia), Gefitinib (indicated for certain breast and lung cancers), and Erlotinib (indicated for non-small cell lung carcinoma and pancreatic cancer).

"Cytokine" refers to a small protein involved in cell signalling and includes chemokines, interferons, interleukins, tumor necrosis factors.

"Low molecular weight inhibitor" as applied to a gene refers to a nucleic acid molecule configured to inhibit expression of a specific target gene. Examples include siRNA, shRNA, miRNA, antisense oligonucleotides, and ribozyme molecules. Small inhibitory RNA (siRNA) are small double stranded RNA molecules which induce the degradation of mRNAs. Micro RNA's (miRNAs) are single stranded (∼22nt) non-coding RNAs (ncRNAs) that regulate gene expression at the level of translation. Alternatively, small hairpin RNA (shRNA) molecules are short RNA molecules having a small hairpin loop in their tertiary structure that may be employed to silence genes. The design of miRNA or shRNA molecules capable of silencing a specific gene will be apparent to those skilled in the field of miRNA or shRNA molecule design. As an alternative, the level of expression of a specific target gene can be modulated using antisense or ribozyme approaches to inhibit or prevent translation of mRNA transcripts or triple helix approaches to inhibit transcription of the specific gene. Antisense approaches involve the design of oligonucleotides (either DNA or RNA) that are complementary to mRNA for the gene. The antisense oligonucleotides will bind to the complementary mRNA transcripts and prevent translation. Ribozyme molecules designed to catalytically cleave mRNA transcripts from a specific gene can also be used to prevent translation and expression. (See PCT International Publication WO90/11364, published October 4,1990 ; Sarver et al. , 1990, Science 247: 1222-1225). As an alternative, the level of expression of a specific target gene can be modulated (increased) using small activating RNA (saRNA).

"Specific for the disease" means that the therapeutically active agent has therapeutic activity against the disease. Thus, when the disease is cancer, the therapeutically active agent may be an anti-cancer drug capable of being expressed by a transformed phagocytic cell (for example an anti-cancer peptide or monoclonal antibody), or an agent capable of re-programming the phagocytic cell to anti-cancer phenotype (for example by inhibition of the NFκB pathway.

Typically, the cancer is selected from the group comprising: esophagogastric cancer; fibrosarcoma; myxosarcoma; liposarcoma; chondrosarcoma; osteogenic sarcoma; chordoma; angiosarcoma; endotheliosarcoma; lymphangiosarcoma; lymphangioendotheliosarcoma; synovioma; mesothelioma; Ewing's tumor; leiomyosarcoma; rhabdomyosarcoma; colon carcinoma; colorectal carcinoma; pancreatic cancer; breast cancer; ovarian cancer; prostate cancer; squamous cell carcinoma; basal cell carcinoma; adenocarcinoma; sweat gland carcinoma; sebaceous gland carcinoma; papillary carcinoma; papillary adenocarcinomas; cystadenocarcinoma; medullary carcinoma; bronchogenic carcinoma; renal cell carcinoma; hepatoma; bile duct carcinoma; choriocarcinoma; seminoma; embryonal carcinoma; Wilms' tumor; cervical cancer; uterine cancer; testicular tumor; lung carcinoma; small cell lung carcinoma; bladder carcinoma; epithelial carcinoma; glioma; astrocytoma; medulloblastoma; craniopharyngioma; ependymoma; pinealoma; hemangioblastoma; acoustic neuroma; oligodendroglioma; meningioma; melanoma; retinoblastoma; primary and metastatic tumors, and leukemias. Typically, treatment of the cancer entails reducing one or more of survival, proliferation and migration of, or invasion by, cancer cells.

### MATERIALS AND METHODS

### Generation of bacterial vectors

*E.coli* K12 MG1655 was used in all experiments (UCC culture collection). For *in vitro* work a GFP plasmid (pMAX-GFP, Amaxa, USA) under the control of a eukaryotic promoter was introduced into this strain by electro-transformation. For optimal transfection efficiency the hlyA gene was introduced into bacteria also². Where relevant, heat-killing of the bacteria was performed by heating at 95° C for 1 hour. For all *in vivo* work, a *hlyA*-expressing *E. coli* MG1655 strain (referred to as *E. coli* hereafter) was employed; MG1655 was transformed with a *hlyA* plasmid (pNZ44 - generated by Dr. Joanne Cummins, Cork Cancer Research Centre). The mCherry-bearing plasmid (Takara Bio Europe/Clontech, France) is commercially available. A plasmid bearing a FLAG-tagged protein (a kinase-negative mutant of IKK2) was a kind gift from Dr. Rainer de Martin³.

*E. coli* was transformed with the therapeutic plasmid carrying a kinase-negative mutant of IKK2 (IKK2-DN) - a kind gift from Dr. Rainer de Martin⁷.

GFP and IKK2-DN genes were driven by eukaryotic promoters. Presence of the *hlyA* and therapeutic plasmid in the bacteria was confirmed by colony PCR to detect the *hlyA* and ampicillin resistance genes respectively.

### Preparation of bacteria for in vitro and in vivo gene delivery

The appropriate bacterial stock was inoculated into 10 ml LB-broth with antibiotic selection and grown overnight (∼16 H) at 37°C, shaking at 200 rpm. The optical density of the culture was measured at a wavelength of 600 nm (OD₆₀₀) and the culture then diluted to an OD₆₀₀ = 0.1. The culture was grown for a further 2-3 H at 37°C, shaking at 200 rpm and the bacteria harvested when the OD₆₀₀ reached 0.6-0.8. 1 ml culture was then pelleted by centrifugation (13500 rpm, 1 min), the pellet was washed twice in PBS and resuspended in 1 ml PBS. The concentration of this solution had previously been determined to be approximately 1x10⁹ CFU/ml. This stock solution was then diluted in PBS to the appropriate concentration.

### Bacterial gene delivery to RAW 264.7 macrophages

RAW 264.7 cells were obtained from American Type Culture Collection (ATCC, Manassas, VA) and maintained in DMEM medium (Sigma-Aldrich) supplemented with 10% foetal bovine serum (FBS) (Sigma-Aldrich) in a 37°C incubator with 5% CO₂. For experiments, 6 x10⁵ cells were seeded per well of a 6-well plate in 2ml media. Cell adherence and a spreading morphology were confirmed using a light microscope. After 24 H the media was changed before the cells were used for further experimentation. Bacteria carrying pMAX-GFP were prepared as above and added directly to the RAW 264.7 macrophages at different multiplicities of infection (MOI). Cells and bacteria were co-incubated for 5 H, after which the bacteria were removed and the cells were washed twice with DMEM containing 1% Penicillin and Streptomycin (Sigma-Aldrich). The cells were allowed recover in antibiotic-supplemented medium at 37°C for a further 22 H prior to analysis. The efficacy of gene delivery was indicated by both fluorescent microscopy using an inverted Nikon Eclipse fluorescent microscope (Mason Technology) and also by the number of GFP⁺ cells as determined by flow cytometric analysis following cell preparation; the adherence of the cells to the plate was broken by incubating in Trypsin- EDTA for 5 min, gently scraping and pipetting up and down. A cytotoxicity assay was performed to assess cell viability in response to each of the experimental conditions using the Nucleocounter system (ChemoMetec, Bioimages Ltd). Cells were resuspended in PBS with 10% FBS to prevent clumping prior to analysis. A FACSLSRII 5 laser (UV/violet/blue/yellow-green/red) cytometer and BD Diva software (Becton, Dickinson) were used to measure GFP positivity. For each sample, 50-100,000 events were recorded. Dead cells/debris were gated out of the analysis based on their low FSC and high SSC characteristics and autofluorescence was controlled by the appropriate gating controls. The proven gene transfer vector Lipofectamine 2000 (Invitrogen) was used as a positive control.

### Tumour induction and bacterial delivery in vivo

For the mouse CT26 colon tumour model (ATCC); 6-8 week old female specific-pathogen-free Balb/C mice, weighing 17 to 20g (Harlan UK), were anaesthetised and the minimum tumourigenic dose (1x10⁵ cells) was injected subcutaneously (s.c.) into their shaved right flank. 18 days post tumour induction (average tumour volume of 100 mm³), the mice (n =2-3 per group per time point) were injected intra-tumourally (i.t.) at three different sites with a total dose of 1x10⁶ CFU of bacteria in a 50 µl volume using a 32 gauge needle.

The ID-8-Fluc cell line was a kind gift from Dr. Kah Whye Peng, Mayo Clinic, USA. For the mouse ID8 ovarian cancer model, 6-8 week old albino C57BL/6 mice (Harlan, UK) had their peritoneal cavities primed by injecting 50 µl of pristine intraperitoneally (i.p.) (Cosmo Bio Co., Ltd., China). One week later 1 x 10⁶ luciferase-expressing mouse ovarian surface epithelial ID8 cells were injected directly into the peritoneum leading to the formation of multiple tumours and ascites within 8 weeks (data not shown). For assessment of bacterial gene delivery 1x10⁶ CFU of bacteria were injected i.p. in a 100 µl volume using a 28 gauge needle (n =2-3 mice per group per time point).

All mice were housed in a conventional environment (temperature 21°C, 12 h light: 12 h darkness, humidity 50%) in a dedicated animal holding facility. They were fed a standard non-sterile pellet diet and tap water ad libitum. Mice were allowed 2 weeks to acclimatize before entering the study. All animal procedures were performed according to national ethical guidelines following approval by the University College Cork Animal Experimentation Ethics Committee.

### Detection of FLAG from in vivo samples

A plasmid bearing a FLAG-tagged protein (IKK2-DN) was used as a reporter for detection of gene delivery within solid CT26 tumours. The tumours (n= 2 per group) were harvested and a single cell suspension prepared as follows; the tumour mass was incubated in 1 ml collagenase (Sigma) and dispase (Sigma) buffer (3 mg/ml of each) at 37°C in a shaking incubator for 10 min. The sample was then mixed further by pipetting through 10 ml and then 5 ml pipettes, before passing through a mesh filter in a physical extrusion technique. The cells were pelleted by centrifugation (1500 rpm for 5 min at 4°C), washed twice in PBS and resuspended in 2 ml red cell lysis buffer (Sigma) with 5 min incubation at room temperature. 30 ml media with serum was added to stop the lysis process. Cells were recovered by centrifugation again, washed once in PBS and counted - both viable and non-viable. 1x10⁶ viable cells were aliquoted per sample to be analysed and cells were fixed and stained. The following antibodies and test concentrations were used; anti-F4/80-PE (clone BM8) (eBioscience, 0.2 µg/test), anti-CD68-PerCP/Cy5.5 (clone FA-11) (Biolegend, 0.25 µg/test) and anti-FLAG Alexa Fluor 647 (Cell signalling, 1 in 450 dilution).

### Analysis of in vivo cytokine profile

A representative portion of tumour (approximately one third) was harvested from animals, flash frozen and stored at -80°C. Cytokine analysis was performed on a mouse pro-inflammatory multi-plex plate (MSD) as per the manufacturer's instructions. The following preparatory work was performed on tumour samples; samples were thawed on ice and a 300 mg specimen was isolated. The appropriate volume of homogenisation buffer (PBS with protease inhibitor cocktail [Roche]) was added to each specimen to yield a concentration of 100 mg of tissue per ml and a uniform solution achieved using a tissue homogeniser. 500 µl of this solution was centrifuged at 1000g and the supernatant recovered. The supernatant was diluted 1 in 20 and 50 µl (equivalent to 250 µg of tissue) added per well.

### Monitoring tumour progression

Tumour volume was determined from measurements of the tumour in two dimensions using a callipers and the formula V=ab²π/6, where a is the longest diameter of the tumour and b is the longest diameter perpendicular to diameter a. Measurements were taken on alternate days from when the tumour first emerged until death of the animal. They were also taken immediately prior to culling the animal for T_{Reg} quantification.

### Histology

Histology was used to detect mCherry gene delivery to solid CT26 tumours. Tumours were removed, halved and snap-frozen in optimal cutting temperature compound (Tissue-Tek; Sakura Finetek) using liquid nitrogen. Frozen tumours were cryosectioned (5µm), fixed for 5 min in ice-cold acetone-ethanol (3:1 ratio), and blocked with blocking serum for 45 min at room temperature in a humidified chamber. Blocked sections were stained with purified rat monoclonal F480 antibody (Clone: CI:A3-1, Abcam) and counterstained using goat anti-rat Alexa Fluor 488-conjugated anti-IgG antibody (Invitrogen, Biosciences Ltd). Stained sections were mounted in ProLong Gold antifade reagent (Invitrogen) and visualized using a fluorescence microscope (Olympus BX51) and Olympus DPX software. mCherry was visible using the Texas red filter of the microscope.

### Flow cytometry

Flow cytometry was used to detect mCherry gene delivery within ID8 ascites and to assess vector delivery selectivity and vector-mediated recruitment of phagocytic cells. Approximately 1ml ascites fluid was harvested from the peritoneum of each animal via a 23- gauge needle and kept on ice until processing. Cells were recovered from the ascites by centrifugation at 1500rpm for 5 min, washed twice in PBS and subjected to red cell lysis buffer (Sigma) for 5 min. Media with serum was added to stop lysis. Cells were recovered by centrifugation again, washed in PBS and viable and non-viable cells were counted. 1x10⁶ viable cells were aliquoted per sample to be analysed and fixed in 4% paraformaldehyde. Cells were then permeabilized, stained intracellularly with an unconjugated monoclonal rat anti-mCherry antibody (Life technologies, 0.5 µg/test), and counterstained with a donkey anti-rat Alexa Fluor 488-conjugated anti-IgG antibody (Molecular probes, dilution 1:300). Relative fluorescence intensities were measured using a FACSLSRII 5 laser (UV/violet/blue/yellow-green/red) cytometer and BD Diva software (Becton, Dickinson). For each sample, 100,000 events were recorded. Dead cells/debris were gated out of the analysis based on their low FSC and high SSC characteristics. Background staining was controlled by the use of FMO (fluorescence minus one) controls. The results represent the percentage of positively stained cells in the total cell population exceeding the background staining signal. The specificity of vector delivery was determined by gating on the mCherry⁺ cells and identifying cell types using the following antibodies; anti-F4/80-PE (Clone: BM8, Biolegend), anti-CD11c-PE-CY7 (Clone: HL7, BD Pharmingen) and anti-LY6G/6C-APC-CY (Clone: RB6-8C5, Biolegend). Macrophages were defined as F480⁺, DCs were defined as CD11C⁺, neutrophils were defined as Ly6G⁺, T cells were defined as CD3⁺ and identified using anti-CD3 AF700 (Clone: eBio500A2, eBioscience) antibody, NK cells were defined as DX5⁺ and identified using anti-CD49b- Pe-CY7 (Clone DX5, Biolegend) and NK T cells were defined as CD3⁺DX5⁺.

### Statistical analysis

Data are presented as the mean ± the standard error of the mean (SEM) unless otherwise stated. All statistical tests were performed using commercially available statistical software (GraphPad Software Inc.). *In vitro* experiments were performed with a minimum of 3 replicates per group. *In vivo* experiments were performed with a minimum of 3 mice per group. Unless otherwise stated statistical significance was determined by unpaired Student's *t* test. A *p* value of 0.05 was considered significant.

### RESULTS

### E. coli MG1655 mediates gene delivery to phagocytic cells in vitro

RAW 264.7 mouse macrophage cells were exposed to the non-invasive bacterium *E. coli* MG 1655 bearing a GFP plasmid under the control of a eukaryotic promoter. After 5 H exposure and a further 22 H to allow macrophage recovery and protein expression, the number of GFP⁺ cells was determined by flow cytometry (Fig. 1). The lower MOI's of 3 and 30 did not yield any GFP positivity, while at an MOI of 300 the mean transfection efficiency and resulting GFP positivity of the viable cells approached 50%. The equivalent amount (MOI 300) of heat-killed GFP⁺ bacteria or plasmid DNA did not yield GFP positivity. Although mammalian cell death was observed in response to high numbers of bacteria (Suppl. Fig. 1), GFP expression was measured only within the viable cell population as determined by their FSC/ SSC characteristics. Gating on the non-viable cell population yielded negligible GFP positivity. While lipofection resulted in detection of fewer transfected cells than bactofection at MOI 300, resulting transgene expression was much higher as evidenced by brighter fluorescence within lipofected cells on both FACS and microscopic analyses.

### E. coli MG1655 mediates gene delivery to phagocytic cells in vivo

Bacterial delivery to macrophages *in vivo* was examined, using the mouse colon cancer CT26 subcutaneous tumour model, as a solid tumour provides a readily accessible "organ" where macrophages are known to be relatively abundant. *E. coli* MG1655 bearing a plasmid coding for a FLAG-tagged protein as a reporter were injected directly into CT26 tumours. 48 H later, the tumours were harvested and individual cells analysed for FLAG expression by flow cytometry. To specifically examine macrophages, this population was gated on using CD68 and F4/80. At 48 H, the bacterial vector had not influenced the proportion of macrophages present in the tumour (Fig. 2a). When non-specific binding of the anti-FLAG antibody (Suppl. Figure 2) was controlled for, transfection efficiency of the gated population was observed at ∼20% *in vivo.* Although the anti-FLAG antibody displayed high non-specific binding (Suppl. Fig. 2), the increased FLAG positivity indicates successful host cell transfection. To interrogate the cell-type specificity of transfection, the number of FLAG⁺ cells among the total tumour cell population was determined to be 17.2% (Fig. 2b). Of these FLAG⁺ cells, 20.6% were identified as double positive macrophages (CD68+, F4/80+). This indicated that ~80% of the FLAG⁺ cells were other cell types, perhaps other phagocytic cells. The identification of these other cell types merited further investigation (see below).

To further confirm bacterial-mediated gene delivery to phagocytic cells *in vivo,* protein expression was examined using an alternative methodology. Immunohistochemistry was employed to detect *E. coli* MG1655-mediated delivery of fluorescent mCherry (Fig. 3). The native mCherry protein was observed to be intra-cellular within macrophages.

### E. coli mediated in vivo gene delivery to phagocytic cells in a murine malignant ascites model

Similar experiments were performed using a non-solid tumour model, the mouse ovarian ID8 peritoneal ascites model. *E. coli* MG1655 bearing a plasmid coding for mCherry were injected directly into the peritoneal cavity of ascitic ID8-tumour bearing mice. Ascitic fluid was recovered from the cavity of different mice at 24, 48 and 72 H post bacterial administration and cells analysed for mCherry expression by flow cytometry (Fig. 4a). No mCherry⁺ cells were detected at 24 H, 10% were mCherry⁺ at 48 H and 50% were mCherry⁺ at 72 H (p < 0.0001, Fig. 4b). FACS controls used to determine the threshold for non-staining cells are presented in Suppl. Figure 3

### Transfected cell type phenotyping

The phenotype of the mCherry⁺ ascitic cells was assessed. Of note, phagocytic cells accounted for 100% of mCherry⁺ cells at both time points, indicating that this gene delivery strategy is specific for phagocytic cells (Fig. 5a). At 48 H, macrophages represented the dominant phagocytic cell type scavenging the bacterial vector, with 96% of mCherry⁺ cells expressing the macrophage marker F4/80, while the remaining 4% were either DCs or neutrophils. However, at the later time point of 72 H, reporter gene expression was similar between macrophages (33%), DCs (40%) and neutrophils (27%) (Fig 5b). FMO controls are presented in Suppl. Fig. 4. Of note, the identity of the mCherry⁺ cells is based on strict appropriation of cell surface marker expression to definitive cell types and is open to interpretation given the high level of plasticity associated with phagocytic populations. Indeed, considerable overlap between surface markers was observed (Fig. 5C).

### Bacterial delivery provokes phagocytic cell differentiation in ascites

Relative to control animals that had not received bacteria, the bacterial vector did not result in a change to the total number of cells per ml of ascites fluid recovered (Fig. 6a). However, the percentage of phagocytic cells (identified as F4/80⁺ macrophages, CD11c⁺ DCs or Ly6G⁺ neutrophils) was increased at every time point following bacterial administration (Fig. 6b and c). This suggested that immature phagocytic cells were differentiating in response to the bacterial vector. At the earlier time points (24 H and 48 H), macrophages were the predominant phagocytic cell representing 40% of the total ascitic cell population. By 72 H relative macrophage abundance had subsided and DCs and neutrophils were the dominant populations in terms of total ascitic cells and phagocytic cells. In order to obtain a more complete picture of the host immune response to the bacterial vehicle, the percentage of lymphocytes and NK cells in ascitic fluid was analysed at 48 H post bacterial administration, and it was found that the majority of non-phagocytic cells present were CD3⁺ T lymphocytes (Fig. 6d).

### Influence of in vivo therapeutic gene delivery on intra-tumoural cytokine profiles

*E.coli*+/- IKK2-DN encoding plasmid, or PBS was i.t. injected to growing CT26 tumours. The cytokine profile within the tumour was assessed at three time points post treatment. Results are displayed in Fig. 7. Highest differences overall were observed four days post treatment, and statistics for this timepoint are displayed in Fig. 7 (right panel). At day four, significant change in cytokine levels could be attributed to the IKK2-DN gene. Cytokine changes invoked within the tumour involved significantly increased (p<0.05) IL-12, IL-1β, IL-6 and mKC, which in all cases is consistent with a pro-inflammatory response. Overall, these data demonstrate that, with the exception of an increase in mKC at day 2 and day 7, the bacterial vector had a minimal effect on the intra-tumoural cytokine profile. In contrast the bacterial vector carrying IKK2-DN induced a significant change in the cytokine profile (p < 0.05) within the tumour.

### References

1. Hagemann T, Lawrence T, McNeish I, Charles KA, Kulbe H, Thompson RG,. Balkwill FR. "Re-educating" tumor-associated macrophages by targeting NF-kappaB. *The Journal of experimental medicine* 2008; **205:** 1261.PMID: 18490490
2. Guo H, Zhang J, Inal C, Nguyen T, Fruehauf JH, Keates AC, Li CJ. Targeting tumor gene by shRNA-expressing Salmonella-mediated RNAi. Gene therapy 2011; 18: 95.PMID: 20811467
3. Oitzinger W, Hofer-Warbinek R, Schmid JA, Koshelnick Y, Binder BR, de Martin R. Adenovirus-mediated expression of a mutant IkappaB kinase 2 inhibits the response of endothelial cells to inflammatory stimuli. Blood 2001; 97: 1611.PMID: 11238099

## Claims

1. A live non-invasive bacterium comprising a transgene under the control of a promotor, wherein the transgene encodes a phagocytic cell-relevant non-immunogenic therapeutically active agent.

2. A live non-invasive bacterium as claimed in Claim 1 in which the phagocytic cell relevant therapeutically active agent comprises an effector molecule capable of re-programming a disease associated macrophage from a pro-disease phenotype to an anti-disease phenotype.

3. A live non-invasive bacterium as claimed in Claim 2 in which the effector molecule is capable of re-programming a tumor-associated macrophage (TAM) from a pro-disease phenotype to an anti-disease phenotype.

4. A live non-invasive bacterium as claimed in Claim 4 in which the effector molecule comprises a macrophage NFκB pathway inhibitor.

5. A live non-invasive bacterium as claimed in Claim 1 in which the phagocytic-cell relevant non-immunogenic therapeutically active agent comprises an effector molecule capable of directly targeting a cancer cell.

6. A live non-invasive bacterium as claimed in Claim 5 in which the effector molecule is a protein, peptide, or a monoclonal antibody.

7. A live non-invasive bacterium as claimed in any preceding Claim in which the promotor is a eukaryotic promotor.

8. A live non-invasive bacterium as claimed in any preceding Claim in which the therapeutically active agent is selected from a protein, a peptide, and a low molecular weight gene expression inhibitor.

9. A live non-invasive bacterium as claimed in any preceding Claim, **for use** in a method of treating a disease in a mammal **characterised by** phagocytic cell accumulation at a disease locus in the mammal, wherein the phagocytic cell-relevant therapeutically active agent is specific for the disease.

10. A live non-invasive bacterium as claimed in Claim 9, **for use** of Claim 9, in which the disease is cancer, and wherein the phagocytic cell-relevant therapeutically active agent comprises an effector molecule capable of re-programming a tumor associated macrophage (TAM) from a pro-tumorigenic phenotype to an anti-tumor phenotype.

11. A live non-invasive bacterium as claimed in Claim 10, **for use** of Claim 10, in which the effector molecule is a low molecular weight inhibitor of macrophage NFκB pathway.

12. A live non-invasive bacterium as claimed in Claim 11, **for use** of Claim 11, in which the disease is cancer, and wherein the phagocytic cell-specific therapeutically active agent comprises an effector molecule capable of directly targeting a cancer cell.

13. A live non-invasive bacterium as claimed in Claim 12, **for use** of Claim 12, in which the effector molecule is a monoclonal antibody.

14. A live non-invasive bacterium as claimed in any of Claims 10-13, **for use** of any of Claims 10-13, in which the cancer is a solid tumor cancer, and in which the live non-invasive bacteria is administered intratumorally.

15. A live non-invasive bacterium as claimed in any of Claims 10-13, **for use** of any of Claims 10-13, in which the cancer is a peritoneal cancer, and in which the live non-invasive bacteria is administered intraperitoneally.
